# EUROPEAN PATENT APPLICATION

(11) **EP 1 304 079 A2**
(43) Date of publication of application: **23.04.2003**
(21) Application number: 03075212.5
(22) Date of filing: 11.08.2000
(51) Int. Cl.: A61B 17/128

(54) **Applicator for surgical clips**

(30) Priority: 14.08.1999 GB 9919170
(62) Divisional of application: 00953301.9
(71) Applicant: Femcare (Cyprus) Ltd., Limassol, Cyprus (CY)
(72) Inventor: Giddins, Darren John, Aspley, Nottingham NG8 5SD (GB); Sadler, Kenneth George, Horsham, West Sussex RH12 1JN (GB)
(74) Representative: Baker, Colin John

(57) **Abstract**

The applicator comprises an operating handle and associated barrel which may contain a plurality of surgical clips which may be induced along the barrel and sequentially fed to an operating end while the clips may be closed by pressure exerted by movement of the inner section of the barrel against an outer section.

## Description

The present application relates to applicators for surgical clips and more particularly to applicators for ligation clips.

Certain known applicators for clips use a push rod and associated link mechanism to cause rotation of an articulated jaw to produce compression forces on a clip and to thereby compress the clip causing it to close preferably with a latching action. The compression forces on the relatively slender central rod produce the risk of buckling with consequent lost motion. In addition, the mechanical force from the link causes radial reaction forces and consequent friction at the interface between the push rod and the outer tube.

For known applicators, this creates a requirement that the applicator must be made from high grade components which will withstand such forces and be able to perform many clip closure operations.

Additionally such designs with rotational closure forces usually require a clip to be inserted into the jaws of the applicator prior to clip closure. Thus, the applicator can only apply one clip at a time.

EP-A-820725 discloses a surgical instrument for performing anastomosis of tubular body tissues and comprises inner and outer members which move relative to each other to compress a clip by compressing a force on the clip in a direction parallel to the relative movement direction of the inner and outer members.

EP-A-916309 discloses with particular relevance to Figure 8A an applicator for a clip and compresses inner and outer members which can move relative to each other, the clip being positioned between jaw members attached to the inner and outer members and the clip being closed by relative movement the force applied to the clip being in parallel with the relative movement direction of the inner and outer members.

WO-A-9108708 discloses a suture tie device system which effects closure of a suture tie by relative movements between inner and outer members pressure being exerted on the suture tie in a direction parallel to the relative direction of movement of the members.

It is an object of the present invention to provide an applicator for surgical clips which is of a simpler, more robust design to such known applicators.

It is a further object of the preferred embodiment of the present invention to provide an applicator which can store and be used to apply a multiplicity of clips.

Such an applicator may be partially or completely disposable.

The present invention therefore provides an applicator for surgical clips having upper and lower jaw members with co-operating locking means when in a closed position, said applicator comprising elongate tube means, said elongate tube means including means for attachment to an operating handle, said elongate tube means comprising an inner member and an outer member, said inner and outer members being slidable relative to each other, said inner member and said outer member being shaped to retain at least one surgical clip, said handle being operative to slide said inner member relative to said outer member, said inner and outer members having co-operating surfaces to cause said surgical clip to be forced from an open to a closed position on operation of said handle to slide said inner and outer members relative to each other said sliding motion creating a compressed force, orthogonal to said direction of sliding, between said outer member and said inner member sufficient to compress said upper jaw member and to cause said upper jaw member to lock into engagement with said lower jaw member.

Preferably, said outer member comprises an elongate tube shaped at an end distal from said handle to retain said surgical clip.

Preferably, said shape of said distal end of said outer member comprises an angled sloping platform on which said surgical clip rests, said sloping platform also comprising a raised flange forming stop means for said surgical clips.

Preferably, said inner member comprises an elongate inner member, said elongate inner member comprising means for storing said multiplicity of surgical clips.

Preferably, said application comprises means for indexing said multiplicity of surgical clips down said elongate inner member.

Said elongate inner member preferably includes stop means operable to provide opposing mechanical forces to oppose the force on a surgical clip by said outer member to create closure of the clip.

The present invention also provides a magazine storage system for an applicator for surgical clips having upper and lower jaw members with co-operating locking means when in a closed position, said magazine storage system comprising a plurality of surgical clips stored in a single column array within an elongate tube, said elongate tube comprising means at a proximal end thereof for attachment to a handle, said elongate tube means comprising an inner member and an outer member, said inner and outer members being slideable relative to each other, said inner and outer members being shaped to retain said plurality of surgical clips and said inner and outer members having at a distal end thereof co-operating surfaces shaped to cause said surgical clip to be forced from an open to a closed position when said inner and outer members are moved relative to each other said sliding motion creating a compressed force, orthogonal to said direction of sliding, between said outer member and said inner member sufficient to compress said upper jaw member and to cause said upper jaw member to lock into engagement with said lower jaw member.

Preferably the magazine includes spring means positioned within said inner member, said spring means being operative to urge said plurality of surgical clips from said proximal to said distal end of said elongate tube.

The outer member preferably comprises at the distal end a ledge shaped stop means to arrest movement of said plurality of surgical clips, said ledge shaped stop means providing leverage to effect closure of said clip when said outer member is moved relative to said inner member.

Preferably a ratchet stop means is provided in said inner tube, said ratchet stop means being positioned between said spring means and said plurality of surgical clips. Said ratchet stop means being moveable by said spring means in a direction from said proximal to said distal end of said elongate tube, said ratchet stop means being co-operative with a corresponding ratchet means on said inner member to be prevented from movement in the opposite direction.

Embodiments of the present invention will now be described, by way of example with reference to the accompanying drawings, in which:-
Figure 1 shows the applicator of the present invention in side elevation,
Figure 2 shows the applicator of Figure 1 in cross-sectional elevation,
Figure 3 shows the applicator of Figure 2 in plan view,
Figure 4 shows the barrel portion of the applicator of Figure 2 in plan view,
Figure 5 shows the barrel portion of the applicator of Figure 2 in cross-sectional elevation,
Figure 6 shows the handle portion of the applicator of Figure 2 in cross-sectional side elevation,
Figure 7 shows the handle portion of Figure 6 in end view,
Figure 8 shows the handle section of Figure 6 in cross-section along line A¹-A¹.
Figure 9 shows in detail a portion of the barrel section of the applicator in greater detail, and
Figure 10 shows the barrel of Figure 9 in end view;
Figure 11 shows the barrel portion of the applicator in an exploded view;
Figure 12 shows the handle portion of the applicator in partial cross-section showing the spring actuated first and second levers;
Figure 13 shows schematically a perspective view of the barrel assembly,

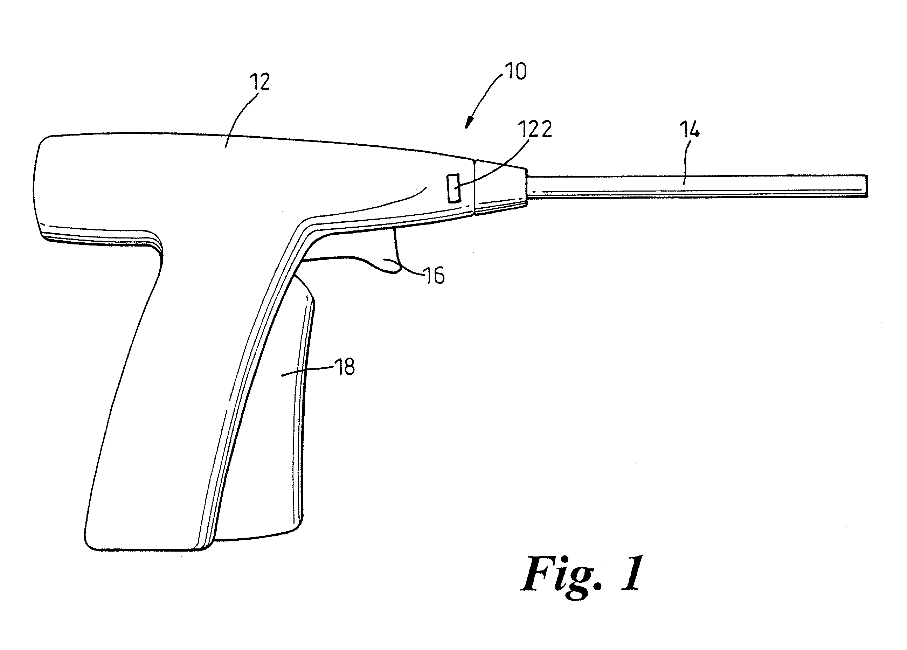

With reference now to the drawings, Figure 1 shows a surgical clip applicator according to the present invention.

The applicator 10 comprise a handle portion 12 and a barrel portion 14. Within the handle portion 12 two levers 16 and 18 are located. The lever 16 in operation indexes the clips down the barrel portion 14 as explained hereinafter and the lever 18 provides the operating force to effect closure of the clip.

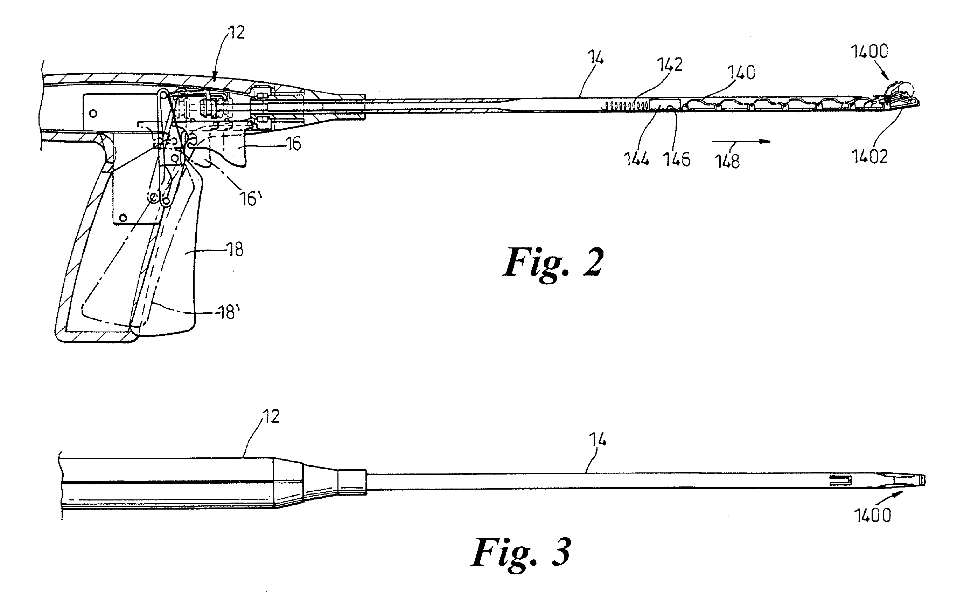

The mechanical detail of the handle portion 12 and the barrel portion 14 are shown in Figure 2.

The lever 16 is shown in two positions 16 and 16'. In position 16', the clips 140 in barrel section 14 are fed forwardly as will be explained hereinafter.

The lever 18 is also shown in two positions, 18 and 18'. In position 18', the lever 18 is operative to provide the pressure to close one of the clips 140.

The lever 16 is also operative in combination with spring 142 (shown only diagrammatically) to move a stop 144 along barrel 14. This stop means provides a stop to enable each clip 140 to be subjected to closing pressure as now described. The stop means is provided with ratchet means 146 to prevent rearward movement whilst allowing forward movement in the direction of arrow 148.

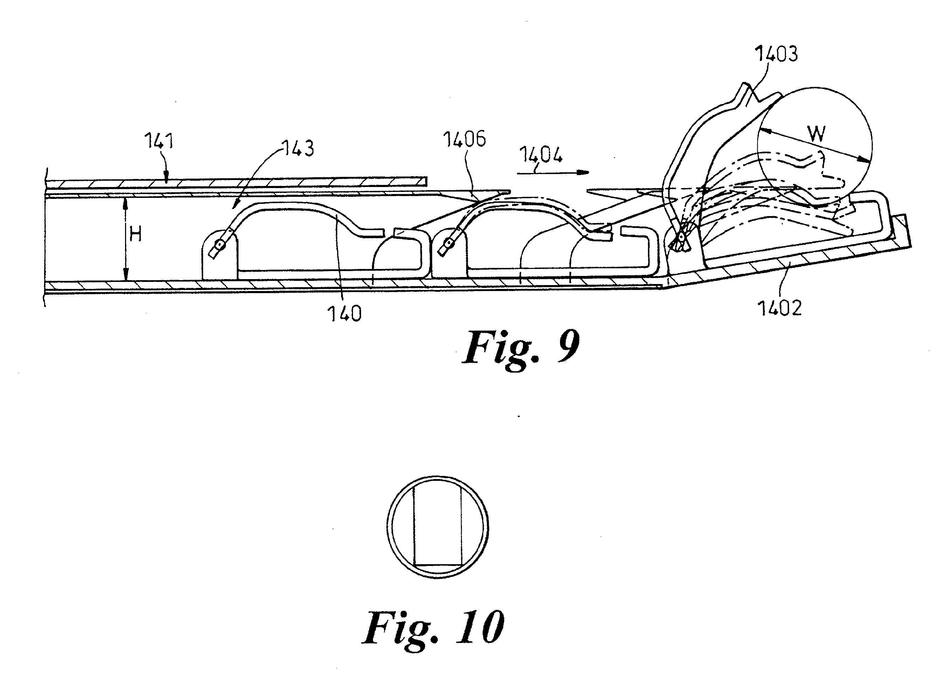

At the end of barrel 14, an operating tip portion 1400 is formed. This comprises an inclined platform 1402 shown in greater detail in Figure 9.

In operation, the lever 18 shown moved to position 18' moves the inner barrel 143 in the direction of arrow 1404. The clips 140 are closed by compression between the lower platform 1402 and the inner barrel end portion 1406 as shown in the segmental drawing in Figure 9.

The barrel section 14 may be disposable and may be loaded with a multiplicity of clips eg 2, 4, 6, 8 or 12 dependent on the operation being performed.

The barrel section 14 has a diameter of H and as can be seen by the circle, the clip when it emerges from the barrel into the end section 1402 has a jaw width W. This defines the width to which the jaw will open, taking into account the silicon rubber lining 1403. It may be seen that with this design of applicator and clip the jaw opening W is substantially larger than the diameter H thereby providing a wide capture width.

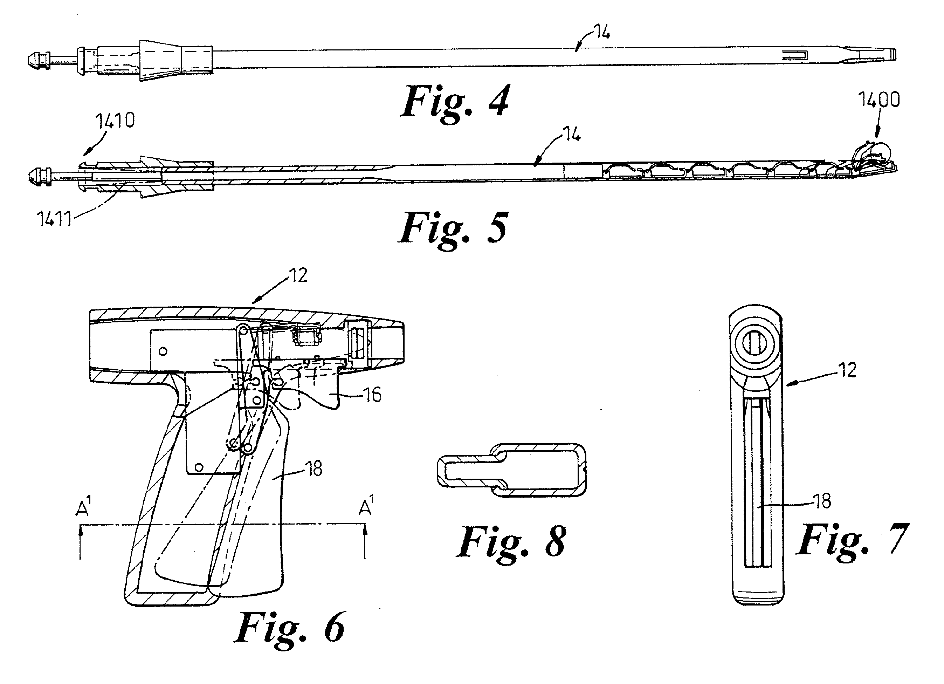

With reference to Figures 4 and 5, the barrel section is shown in isolation from the handle 12. The handle lever 18 operates on shoulder 1410 to move inner tube 143.

Handle portion 12 is shown in greater detail in Figures 6 to 8.

The handle portion 12 may be disposable or may be reusable but preferably the barrel section 14 is disposable and is supplied in a "sterilised form for each operation".

The barrel portion 14 is preferably circular in cross-section. The barrel 14 may be rotated to always be in a suitable orientation by the surgeon so that the handle 12 may always be held in a suitable position allowing easy operation of lever 18.

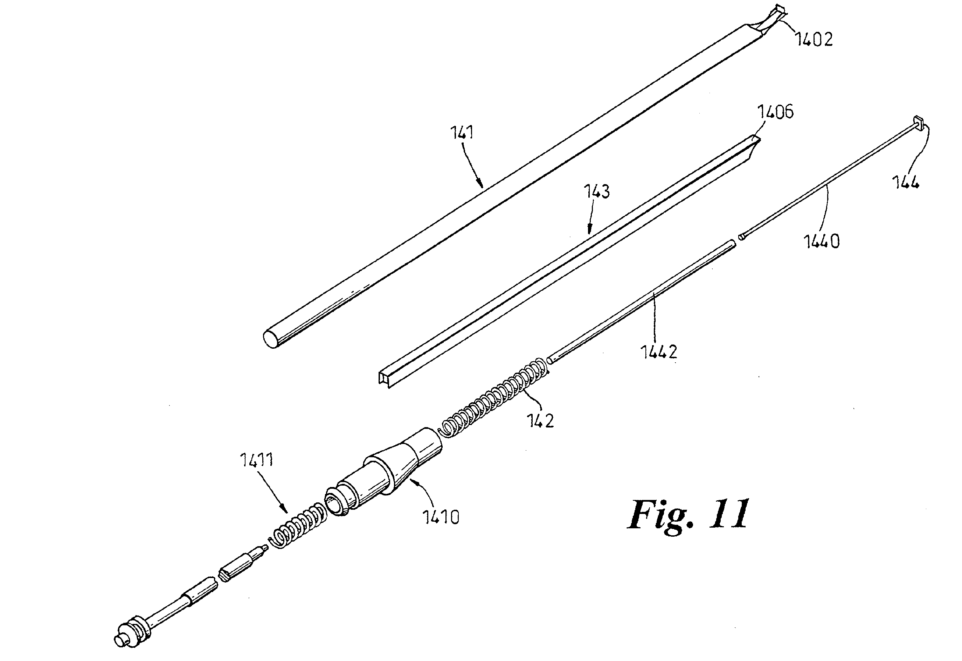

With reference now to Figure 11, a preferred example of the barrel portion 14 is shown in exploded form.

The clips 140 are not shown for ease of illustration but will normally be present as shown in previous figures.

The barrel portion 14 comprises an outer tubular portion 141 which comprises a generally circular cross-section tube with the end operational portion 1402 formed integrally at the distal end.

Within the tube 141 is the inner tube 143 which also forms a magazine to house the clips 140. The inner tube 143 is formed integrally with the operational closure means 1406.

Within the magazine section 143 the spring 142 operates against the clip buffer section 144 which in this case comprises a push rod 1440 within a small bore tube 1442.

The barrel sections 141, 143 are located in the shoulder section 1410 which as described is replaceably locatable in the handle 18. The barrel section is spring loaded into the handle by spring means 1411 to ensure correct fitment.

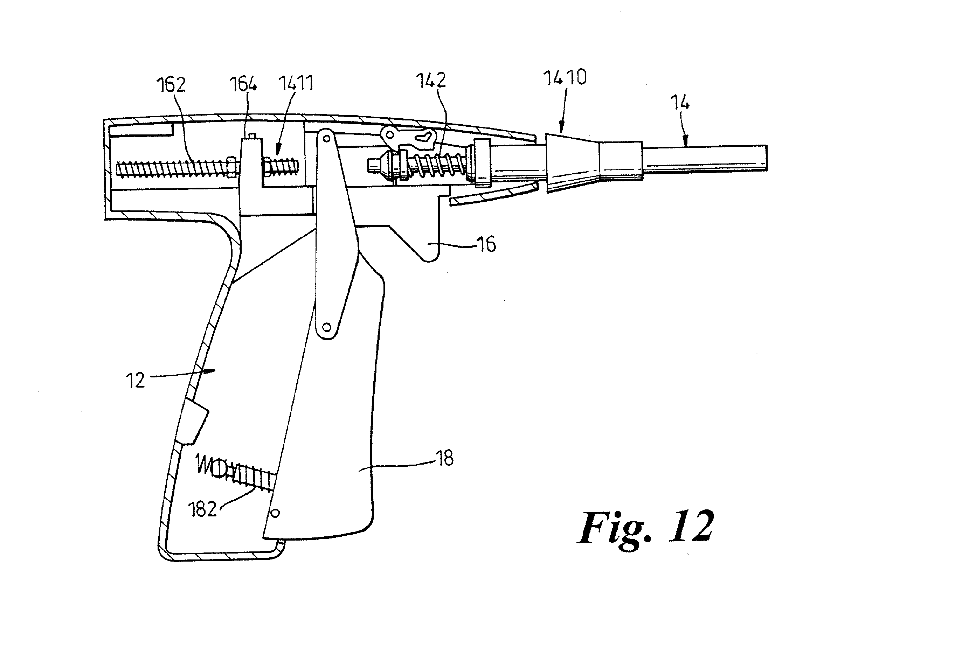

Figure 12 shows the handle 12 of the applicator in partial cross-section illustrating the spring mechanisms.

Parts are numbered as in previous figures.

The main operating lever 18 is spring loaded by a spring 182 to its non-operating position as shown.

The second operating lever 16 is spring loaded by a spring 162 acting against flange member 164 to return lever 16 to its non-operating position.

Since all barrel 14 members are of circular cross-section they are located in handle 12 in a manner enabling the barrel 14 to be rotated with respect to handle 12 allowing the surgeon to selectively orientate the barrel and hence the clip closure with respect to the handle.

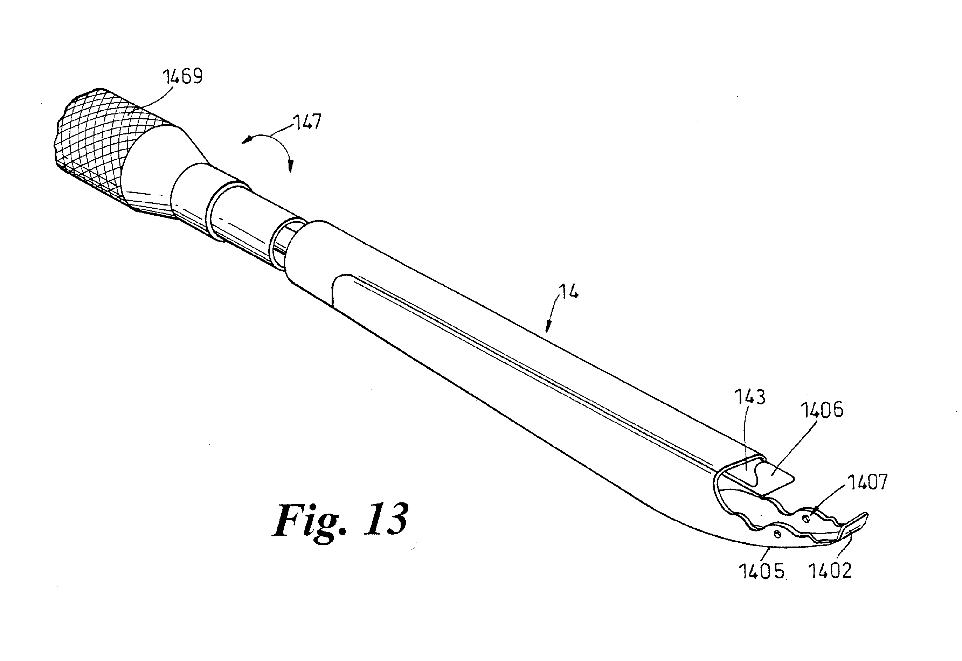

With reference now to Figure 13 the barrel 14 is shown schematically. This figure illustrates two features. The first feature is the change from rectangular section duck tube 143 to a round section.

The other is the optional clip retention feature 1405, 1407 which provides a side grip on the clip to grip the clip by its lower jaw silicone rubber lining.

The superior features of the new applicator design are now summarised.
1. The axial sliding of two components (141; 143) of the shaft is used as a reliable and simple way to transmit actuation force from the operator interface to the clip closing jaws.
2. The outer tube (duck tube) (141) extended to form the clip tray 1403 and jaw forms the tension link. The inner channel 143 is the compression link exploiting the superior compression stability of this member.
3. Within the central core (channel) multiple clips 140 are stored for consecutive advancing and closing.
   The system depends on the relationship between the profile of the upper jaw of the clip, the angle of the end portion 1402 of the outer tube 141 and the profile at the end of the channel (1406) during sliding movement of the two latter items.
   An optional clip retention device (1405, 1407) is included in the clip jaw which grips on the silicone liner at the base of the clip so loss of control is avoided during surgery, but ensures easy release after closure.
   Clip indexing along the channel utilises a one way frictional moving buffer and constant force spring (like staple gun).
   Clip closure is actuated by the large (major) trigger 18 in the handle and clip indexing by the smaller (minor) trigger 16 in the handle.
   The major trigger action and stroke on the applicator is achieved as a result of a large finger bar 18 on the underside of the pistol grip handle to avoid pressure on one finger when closing clips.
   Springs in the handle assembly and clip magazine assembly return the components to the neutral position after clip closure and in readiness for the next clip to be indexed in the clip jaw or tray.
   A single clip system could operate on the same basis but without a long channel to house the clips.
   The handle shape is optimised in relation to ergonomic data.
   The clip magazine may be disposable.
   The sliding tube technique is less likely to cause high localised stress in the tissue due to concentration of deformation at the centre of the upper jaw of the clip.

The design principle of the magazine feed applicator offer the following features.
1. A disposable multi clip magazine.
2. Disposable barrel with clip closure functioning at surgical end but moving in a linear plane rather than through an arc.
3. The barrel and magazine indexed radially so the surgeon can adjust the angle of the jaws in relation to the hand position without changing angle of arm or conversely, if the surgeon has to change his arm position to avoid the patient, then he can still adjust the barrel to the correct working position.
4. A reusable handle with primary trigger for closing the clip, secondary trigger for both moving the clips in the barrel.
5. The handle/barrel arrangement and clip closure mechanism allows the surgeon's hand to remain in constant relationship with the operation of the clip for accurate surgical procedures.
6. The applicator is pre-set at manufacture which avoids calibration and maintenance. Magazines with 2, 4, 6, 12 clips could be preloaded and sold as sterile packs for insertion into common handles in the theatre.
7. The principle could be used for the Filshie clip (British Patent No 2177748) with magazines suitably matched to clip size and common handle. In a preferred embodiment, different colour/identity marking on the magazines would be used to distinguish ligation clips from Filshie clips.
8. The elimination of calibration will reduce the cost of maintenance.
9. The outer barrel of the magazine acts as the tension link and the inner channel as the compression one, so that a clip is held in the clip tray at an angle to the line of force in order to close a clip over a vessel.
10. Clips in the magazine, but not in a position to be closed are preferably retained by small escapement spring in the outer tube and a slot in the channel acting to restrain the clips.
11. In a preferred embodiment, a snap action (audible noise) alerts the surgeon to closure of clips. This avoids excessive pressure on the main trigger mechanism.

A general description of the applicator system and method of operation is as follows:

The clip application system consists of a "pistol" shaped hand grip 12 and a "barrel" 14 that contains the clip magazine. The magazine barrel sterile packs are preferably text marked and colour coded to identify the type and number of clips that they contain. The barrels themselves are colour coded in the same way. These magazine barrels may repeatedly be loaded with single clips, once the internal magazine is exhausted but are intended to be disposable at the conclusion of each procedure. Barrels for hand loading of single clips may be sterilised for repeated use.

The hand grip 12 is common to all barrels and may be sterilised for repeated use. The hand grip incorporates all controls and requires no calibration or adjustment when changing the type of barrel. The operable controls are: a primary trigger which closes and latches clips; a secondary trigger which may prevent inadvertent latching of clips and feeds successive clips into the jaw for use; twin buttons 122 (only one shown in Fig 1) that release the barrel from the hand grip. In addition, a textured grip 1469 (Fig 13) on the rear end of the barrel allows this to be rotated to any convenient orientation relative to the pistol handle.

The handle may be held in either hand, stabilised by the thumb. The secondary trigger 16 is operated by the index finger and the remaining fingers are used to squeeze the trigger 18 . The retainer release buttons 122 are operated by squeezing between the thumb and index finger. All controls require force to be applied in one direction only and return to their normal positions under the action of internal springs.

The principal actions required when using of the applicator system are listed below and described in the following paragraphs.
■ Loading pistol with sterile barrel
■ Loading first clip to jaw
■ Closing/opening clip
■ Latching clip
■ Releasing latched clip and loading next clip
■ Ejecting used barrel from pistol

### Loading pistol with sterile barrel

Barrels (14) are supplied completely enclosed in an individual sterile pack (not shown). If the cylindrical portion of the barrel is gripped in either hand, the part of the pack covering the end to be inserted into the handle may be torn and removed. If the handle is taken up, in either hand, the exposed part of the barrel may be fed into the nose of the handle. When inserted approximately 20mm, a resistance will be felt. Increased insertion force will cause the sprung retainer to retract, allowing full insertion of the barrel. When the barrel is fully inserted, the retainer will engage with an audible click. Proper engagement should be tested by pulling the barrel 14 away from the handle 12. The barrel 14 must be retained but be free to revolve within the pistol 12. The assembled handle and barrel may now be laid down with the sterile sheath in place on the barrel or this may be removed for surgical use and insertion into a cannula.

### Loading first clip to jaw

The pistol 12 is held in either hand, the index finger is placed on the secondary trigger 16 and the remaining fingers are relaxed. The secondary trigger is drawn back to the full extent of travel and then released. A clip 140 will appear at the nose of the barrel and will be carried forward into the jaw. When the secondary trigger is released, the clip will be open.

### Closing/opening clip

The clip 140 may be progressively closed and latched in one continuous action or, if desired, the motion of the applicator may be limited to allow closing the clip but preventing final latching. Holding the pistol in either hand, the primary trigger is squeezed. After a short initial travel, the clip will commence closing and further movement of the trigger to the limit of travel will close and then latch the clip. If the motion is ceased at any point up to latching of the clip and pressure on the trigger is relaxed, the clip will return to the fully open position. The clip is positively retained in the jaw throughout these actions.

If it is desired to positively prevent inadvertent overtravel and consequent latching of the clip, the following procedure is required. With the pistol in either hand and the trigger 18 in relaxed position, the trigger 16 is lightly drawn back. An initial rotational movement will be perceived after which increased resistance will be felt. While maintaining the secondary trigger in this position, the trigger 18 is drawn back. The clip will close as before but further travel beyond the closed position will be prevented as long as the secondary trigger is engaged. When it is desired to allow further travel of the trigger 18 to latch the clip, the trigger 16 is released.

### Latching clip

The clip may be latched by squeezing the trigger (18) to the full extent of travel. As explained above, this may be done directly from the initial position, (clip open), or from the intermediate position, (clip depressed). In all cases, it is important that the trigger 18 be squeezed to the fullest extent of travel once it is desired to latch the clip. After completion of latching, the trigger 18 is released and will return to the relaxed position. The clip will be retained in the jaw.

With reference to Figure 13, the shaped portion of the inner tube 1406 is preferably moved towards the end stop 1402 to effect closure of the clip as shown in Figure 9. This provides the relatively smooth closure action of the clip resulting in uniform distribution of pressure across the tissue being occluded by the clip.

When the clip has been positioned at the end of the barrel in a position to be actuated the portion 1406 of the inner tube contacts the upper jaw of the clip near to the hinge of the clip which will be opened by the pressure of the silicone lining of the clip. Thus, the portion 1406 acts as a stop to prevent the clips from falling out whilst also ensuring maximum occlusion capacity as can be seen in Figure 9.

### Releasing latched clip and loading n-ext clip

When it is desired to release the latched clip, the secondary trigger is drawn back, through the initial rotational movement and into the sliding movement. After a small amount of movement, the clip will be free to leave the jaw. If it is required to engage the next clip, the secondary trigger 16 should be drawn back to the full extent of travel and released. A clip will appear at the nose of the barrel and will be fed forward into the clip tray 1402. When all triggers are released, the clip will be open.

### Ejecting used barrel from pistol

To remove the barrel from the pistol by hand, take up the pistol with the thumb and forefinger placed either side of the body, upon the release buttons 122. If these buttons are squeezed, the barrel may be withdrawn from the pistol. To eject the barrel from the pistol without having to touch the barrel, the pistol is taken in the normal hold and the primary trigger is drawn back to the full extent. The release buttons are then depressed and the barrel will be spring ejected from the pistol. It is important that this action only be performed with the barrel aimed downwards into a suitable waste container (suitably identified for this type of waste).

## Claims

1. An applicator for surgical clips having upper and lower jaw members with co-operating locking means when in a closed position, said applicator comprising elongate tube means (14), said elongate tube means including means for attachment to an operating handle (12), said elongate tube means comprising an inner member and an outer member, said inner and outer members (141, 143) being slidable relative to each other, said inner member and said outer member being shaped to retain at least one surgical clip (140), said handle being operative to slide said inner member relative to said outer member, said inner and outer members having co-operating surfaces (1406, 1402) to cause said surgical clip to be forced from an open to a closed position on operation of said handle to slide said inner and outer members relative to each other said sliding motion creating a compressed force, orthogonal to said direction of sliding, between said outer member and said inner member (1406, 1402) sufficient to compress said upper jaw member and to cause said upper jaw member to lock into engagement with said lower jaw member.

2. An applicator for surgical clips as claimed in Claim 1 in which said outer member comprises an elongate tube shaped at an end (1400) distal from said handle to retain said surgical clip.

3. An applicator for surgical clips as claimed in Claim 2 in which said shape of said distal end of said outer member comprises an angled sloping platform (1402) on which said surgical clip rests, said sloping platform also comprising a raised flange forming stop means for said surgical clips.

4. An applicator for surgical clips as claimed in Claims 1 to 3 in which said inner member (143) comprises an elongate inner member, said elongate inner member comprising means for storing a multiplicity of surgical clips.

5. An applicator for surgical clips as claimed in any one of Claims 1 to 4 in which said application comprises means for inducing said multiplicity of surgical clips down said elongate inner member.

6. An applicator for surgical clips as claimed in Claim 5 in which said elongate inner member includes stop means operable to provide opposing mechanical forces to oppose the force on a surgical clip by said outer member to create closure of the clip.

7. A magazine storage system for an applicator for surgical clips having upper and lower jaw members with co-operating locking means when in a closed position, said magazine storage system comprising a plurality of surgical clips (140) stored in a single column array within an elongate tube, said elongate tube comprising means (1410, 1411) at a proximal end thereof for attachment to a handle (12), said elongate tube means comprising an inner member (143) and an outer member (141), said inner and outer members being slideable relative to each other, said inner and outer members being shaped to retain said plurality of surgical clips and said inner and outer members having at a distal end thereof co-operating surfaces shaped to cause said surgical clip to be forced from an open to a closed position when said inner and outer members are moved relative to each other said sliding motion creating a compressed force, orthogonal to said direction of sliding, between said outer member (1462) and said inner member (1406) sufficient to compress said upper jaw member and to cause said upper jaw member to lock into engagement with said lower jaw member.

8. A magazine storage system for an applicator as claimed in claim 7 including spring means positioned within said inner member, said spring means (142) being operative to urge said plurality of surgical clips from said proximal to said distal end of said elongate tube.

9. A magazine storage system for an applicator as claimed in claim 8 in which said outer member comprises at the distal end a ledge shaped stop means (1402) to arrest movement of said plurality of surgical clips, said ledge shaped stop means providing end stop means to effect closure of said clip when said outer member is moved relative to said inner member.

10. A magazine storage system for an applicator for surgical clips as claimed in claim 9 in which a ratchet stop means (144) is provided in said inner tube (143), said ratchet stop means being positioned between said spring means and said plurality of surgical clips, said ratchet stop means being moveable by said spring means in a direction from said proximal to said distal end of said elongate tube, said ratchet stop means being co-operative with a corresponding ratchet means (146) on said inner member to be prevented from movement in the opposite direction.
